# EUROPÄISCHE PATENTANMELDUNG

(11) **EP 2 105 114 A1**
(43) Veröffentlichungstag der Anmeldung: **30.09.2009**
(21) Anmeldenummer: 09003881.1
(22) Anmeldetag: 18.03.2009
(51) Int. Cl.: A61F 13/06, A61F 5/01

(54) **Stützbandage**

(30) Priorität: 27.03.2008 DE 202008004212 U
(71) Anmelder: Hallufix AG, 81925 München (DE)
(72) Erfinder: Krauss, Axel, 81541 München (DE)
(74) Vertreter: Thoma, Michael

(57) **Zusammenfassung**

Die vorliegende Erfindung betrifft eine Stützbandage, vorzugsweise Metatarsalbandage, zur Gewebestützung an einem Körperglied, bestehend aus einem Bandagegurt mit zwei Gurtenden sowie einem Verschluss zum Schließen des Bandagegurts zu einem Ring. Erfindungsgemäß besitzt der Bandagegurt an seinem einen Endabschnitt einen sich quer zur Gurtlängsachse erstreckenden Durchführschlitz, durch den der andere Endabschnitt des Bandagegurts hindurch geführt ist, wobei der Verschluss zum Schließen des Bandagegurts zu einem Ring zwei an den Innenseiten der Endabschnitte des Bandagegurts angebrachte Verschlussteile zur Befestigung der Endabschnittinnenseiten auf Außenseiten von Bandagegurtabschnitten, die von den Endabschnitten überlappt sind, aufweist. Durch den sich quer erstreckenden Durchführschlitz kommen beiden Bandagegurtenden auf der Bandagenaußenseite zu liegen, so dass beide Bandagenenden gegriffen und einfach auseinander gezogen werden können, um die Spannung in der Bandage fein zu justieren. Ist die gewünschte Spannung erreicht, brauchen lediglich die gespannten Endabschnitte auf die vor der Schlitzdurchführung liegenden Bandagenabschnitte gedrückt werden, die von den genannten Endabschnitten überlappt werden.

## Beschreibung

Die vorliegende Erfindung betrifft eine Stützbandage, vorzugsweise Metatarsalbandage, zur Gewebestützung an einem Körperglied, bestehend aus einem Bandagegurt mit zwei Gurtenden sowie einem Verschluss zum Schließen des Bandagegurts zu einem Ring.

Stützbandagen sind in mannigfacher Form bekannt und unterliegen bisweilen auseinander laufenden Anforderungen. Einerseits soll eine hohe Stützwirkung erreicht werden, was ein bisweilen strammes Anziehen der Bandage erfordert. Andererseits soll die Bandage einen hohen Tragekomfort besitzen, was oftmals eine kompliziertere Gurtführung, die zur feinen Einstellung der Gurtspannung bisweilen verwendet wird, nicht zulässt. Insbesondere Metatarsalbandagen zur Stützung des Mittelfußgewölbes unterliegen hohen Anforderungen an den Tragekomfort, insbesondere wenn diese auch in einem Schuh getragen werden soll, da bei einer derartigen Anwendung die Metatarsalbandage nicht zu dick auftragen sollte und insbesondere keine mehrfachen Hin- und Herführungen besitzen sollte. Herkömmliche Bandagen erfüllen diese auseinander laufenden Anforderungen bislang noch nicht gleichermaßen zufriedenstellend.

Aus der US 1,678,608 ist eine Mittelfußbandage bekannt, bei der der Bandagegurt an einem domförmig gewölbten Druckstück angeschlagen ist, welches einen Druck auf die Fußsohle ausüben soll. Die Anlenkung des Bandagegurtbands an diesem Sohlendruckstück begrenzt den Einsatzbereich der Bandage jedoch auf die Fälle, in denen ein solches Druckstück sinnvoll bzw. wünschenswert ist. Darüber hinaus kann es bei dieser bekannten Bandage durch ungleichförmiges Ziehen von einer Seite her zu einem Verrutschen der Bandage bei ihrer Befestigung kommen. Weiterhin zeigt die US 1,441,907 eine Fußbandage, die S-förmig gleichermaßen um den Mittelfuß und um die Achillessehne herum geschlungen wird. Hierzu umfasst ein Abschnitt der Bandage einen Längsschlitz, durch den ein anderer Gurtabschnitt hindurch geführt ist, so dass sich insgesamt betrachtet eine doppelschlaufenförmige Ausbildung der Bandage ergibt. Die Enden des Bandagegurts sind miteinander vernäht, so dass sich die Stützspannung der Bandage nicht einstellen lässt und allein aus der elastischen Ausbildung des Gurtbandes resultiert.

Aus der US 3,926,186 ist weiterhin eine Knie- und Fußbandage bekannt, die an einem Ende zwei Ösen aufweist, durch die zwei Spannbänder durchgeschlauft und umgeschlagen werden, so dass die umgeschlagenen Enden der Gurtbänder per Klettverschluss an dem noch nicht durchgeschlauften Teil befestigt werden können. Diese Bandage ist jedoch wenig geeignet, um in einem Schuh getragen zu werden, da der stark auftragende Verschluss rasch zu Scheuerstellen führt, soweit die genannte Bandage überhaupt geeignet ist, in einem Schuh getragen zu werden.

Weiterhin ist aus der GB 361,800 eine Fußbandage bekannt, die mit ihren Enden einfach übereinandergeschlagen wird, so dass die einander überlappenden Enden mittels Hakenverschlüssen an dem überlappten, darunter liegenden Gurtabschnitt befestigt werden können. Ein Gurtabschnitt kann hierbei einen Längsschlitz aufweisen, so dass die Bandage in der zuvor genannten Weise zu einer Doppelschlaufe eingeschlagen werden kann, um sowohl den Mittelfuß als auch den Knöchelbereich um die Achillessehne herum einzubandagieren.

Hiervon ausgehend liegt der vorliegenden Erfindung die Aufgabe zugrunde, eine verbesserte Stützbandage der eingangs genannten Art zu schaffen, die Nachteile des Standes der Technik vermeidet und Letzteren in vorteilhafter Weise weiterbildet. Insbesondere soll eine einfach anzulegende, hinsichtlich ihrer Spannung präzise einzustellende Stützbandage geschaffen werden, die einen hohen Tragekomfort bietet und auch innerhalb eines Schuhs bequem getragen werden kann.

Erfindungsgemäß wird diese Aufgabe durch eine Stützbandage gemäß Anspruch 1 gelöst. Bevorzugte Ausgestaltungen der Erfindung sind Gegenstand der abhängigen Ansprüche.

Es wird also vorgeschlagen, die Bandagenenden durch einander hindurch treten zu lassen, so dass durch Auseinanderziehen bzw. Voneinanderwegziehen der Bandagenenden doppelseitig die Gurtspannung fein justiert werden kann, wobei bei Erreichen der gewünschten Spannung diese einfach durch Aufdrücken der Gurtenden auf davon überlappte Gurtabschnitte eingefroren werden kann. Erfindungsgemäß besitzt der Bandagegurt an seinem einen Endabschnitt einen sich quer zur Gurtlängsachse erstreckenden Durchführschlitz, durch den der andere Endabschnitt des Bandagegurts hindurch geführt ist, wobei der Verschluss zum Schließen des Bandagegurts zu einem Ring zwei an den Innenseiten der Endabschnitte des Bandagegurts angebrachte Verschlussteile zur Befestigung der Endabschnittinnenseiten auf Außenseiten von Bandagegurtabschnitten, die von den Endabschnitten überlappt sind, aufweist. Durch den sich quer erstreckenden Durchführschlitz kommen beiden Bandagegurtenden auf der Bandagenaußenseite zu liegen, so dass beide Bandagenenden gegriffen und einfach auseinander gezogen werden können, um die Spannung in der Bandage fein zu justieren. Ist die gewünschte Spannung erreicht, brauchen lediglich die gespannten Endabschnitte auf die vor der Schlitzdurchführung liegenden Bandagenabschnitte gedrückt werden, die von den genannten Endabschnitten überlappt werden.

Die beiden Endabschnitte des Bandagegurts bilden dabei in Weiterbildung der Erfindung zwei voneinander weggerichtete Spannabschnitte, die durch Auseinanderziehen voneinander weg die Stützbandage spannen und in ihrer voneinander weg gerichteten Stellung auf der Außenseite der davon überlappten Bandagegurtabschnitte befestigt werden können. Um einen besonders hohen Tragekomfort auch in Schuhen zu erreichen, ist die Stützbandage vorteilhafterweise frei von Umlenkringen oder vergleichbaren Umlenkvorrichtungen ausgebildet, wobei das Gurtband ohne Zugrichtungsumkehrungen, ohne leporelloähnliche Durchschlaufungen und andere zusätzliche Aufdoppelungen bewirkende Gurtführungsbahnen um das zu bandagierende Körperglied geführt ist. Das Gurtband ist über seine gesamte Länge sozusagen nur einfach gewölbt und lediglich an die Krümmung des zu stützenden Körperglieds angepasst. Hierdurch ergibt sich ein minimales Auftragen der Bandage, was den Tragekomfort beträchtlich erhöht, insbesondere in Schuhen oder unter anderen das jeweilige Körperglied eng umschließenden Vorrichtungen wie beispielsweise Schienen und dergleichen.

Die Verschlussteile, mittels derer die Gurtenden auf den davon überlappten Gurtabschnitten befestigt werden, können grundsätzlich verschieden ausgebildet sein. Beispielsweise kommen Hakenverschlüsse oder Noppen-/Kettenbänder in Betracht. In besonders bevorzugter Ausführung der Erfindung jedoch bilden die genannten Verschlussteile auf den Endabschnittinnenseiten zusammen mit der Außenseite der davon überlappten Bandagegurtabschnitten einen Haken- und Flauschteilverschluss, wie er unter dem Handelsnamen Klettverschluss bekannt ist. Ein solcher Haken- und Flauschteilverschluss beeinträchtigt die Biegsamkeit und Wölbung des Gurtbandes angepasst an die Fußwölbung nicht oder nur minimal, wodurch ein hoher Tragekomfort erreicht werden kann. Zudem ist eine stufenlose Einstellung und Fixierung der Bandagegurtspannung möglich.

Um ein Scheuern der Bandage an einem darüber gelegten Kleidungsstück, beispielsweise einem Socken oder einem Oberschuh zu verhindern und andererseits der Bandage eine bestmögliche Anpassung an die Körpergliedkontur zu geben, ist in Weiterbildung der Erfindung vorgesehen, dass der Hakenteil des Haken- und Flauschteilverschlusses auf der Innenseite der Bandagegurtenden vorgesehen ist, während die Außenseite der hiervon überdeckten Bandagegurtabschnitte als Flauschteil ausgebildet ist bzw. mit einem Flauschteil versehen ist. In vorteilhafter Weiterbildung der Erfindung kann im Wesentlichen die gesamte Bandagegurtaußenseite, ggf. mit Ausnahme der hinter der Schlitzdurchführung liegenden Gurtenden, als Flauschteil ausgebildet sein bzw. mit einem Flauschteilverschluss versehen sein. Hierdurch kann eine maximale Verstellbarkeit der Stützbandage hinsichtlich Bandagedurchmesser erreicht werden, wobei über dem gesamten Verstellbereich eine stufenlose Fixierbarkeit sichergestellt ist.

Um ein vernünftiges Hindurchführen des einen Endabschnitts durch den Durchführschlitz im anderen Endabschnitt zu erreichen, ist in Weiterbildung der Erfindung der Durchführschlitz zumindest so lang wie der hindurchgeführte Endabschnitt breit ist, was wiederum durch eine entsprechend größere Breite des den Durchführschlitz aufweisenden Endabschnitts aufgefangen ist, um diesem Endabschnitt, in dem der quer verlaufende Durchführschlitz ausgebildet ist, seitlich neben dem Durchführschlitz auf beiden Seiten desselben noch genügend Fleisch zu geben. Um dies zu ermöglichen, kann grundsätzlich vorgesehen sein, dass der Endabschnitt, in dem der Durchführschlitz ausgebildet ist, eine Verbreiterung besitzt, so dass darin ein ausreichend langer Durchführschlitz ausgebildet werden kann, durch den hindurch das andere Ende durchgesteckt werden kann. In besonders vorteilhafter Ausführung der Erfindung ist jedoch vorgesehen, dass der durch den Durchführschlitz hindurch geführte Endabschnitt einen Abschnitt verjüngter Breite besitzt, so dass der andere Endabschnitt, in dem der Durchführschlitz ausgebildet ist, auch ohne Verbreiterung auskommt. Hierdurch kann eine bessere Zugspannungsverteilung über den Durchführschlitz hinweg erreicht werden, die ein Zusammenziehen des den Durchführschlitz aufweisenden Endabschnitts oder gar eine Falten- und Wulstbildung verhindert.

Der Abschnitt verjüngter Breite kann grundsätzlich durch ein insgesamt verjüngt auslaufendes Gurtende gebildet sein. In Weiterbildung der Erfindung jedoch wird der Abschnitt verjüngter Breite von einer Einschnürung gebildet, an die in Gurtlängsrichtung zu beiden Seiten hin Abschnitte größerer Breite anschließen, d.h. der Endabschnitt, der durch den Durchführschlitz hindurch geführt ist, ist im Bereich der Hindurchführung sanduhrförmig schmäler ausgebildet und zum Ende hin wieder verbreitert. Hierdurch wird zum einen die gewünschte, faltenfreie Durchführung erzielt, während andererseits an dem hindurch geführten Ende ausreichend Fläche gebildet wird, um ein sicheres Verhaken des Haken- und Flauschteilverschlusses zu realisieren.

Vorteilhafterweise besitzen die Endabschnitte des Bandagegurts im Vergleich zu einem Mittelabschnitt des Bandagegurts eine leicht verringerte Breite, so dass die auf der Außenseite der überlappten Bandagegurtabschnitte liegenden Endabschnitte nicht über die Breite des darunter liegenden Bandagegurts überstehen. Dies erlaubt einerseits ein angenehmes Greifen der Enden beim Spannen, andererseits wird verhindert, dass überstehende Seitenrandabschnitte der Gurtenden, die innenseitig mit einem Hakenverschlussteil belegt sind, neben der Bandage unmittelbar auf die Hautoberfläche drücken.

Die vorliegende Erfindung wird nachfolgend anhand eines bevorzugten Ausführungsbeispiels und zugehöriger Zeichnungen näher erläutert. In den Zeichnungen zeigen:
- Fig. 1:: eine perspektivische Ansicht einer Stützbandage nach einer vorteilhaften Ausführung der Erfindung, wobei die Spannabschnitte bildenden Enden des Bandagegurts im geöffneten, noch nicht fixierten Zustand gezeigt sind,
- Fig. 2:: eine perspektivische, schematische Ansicht der Stützbandage aus Fig. 1 aus einem anderen Blickwinkel, der die Gurtdurchführung von der Bandageninnenseite her zeigt,
- Fig. 3:: eine Seitenansicht der Stützbandage aus den vorhergehenden Figuren, die das Hindurchtreten der Bandagegurtenden verdeutlicht, und

- Fig. 4:: eine Abwicklung des Bandagegurtbandes der Stützbandage aus den vorhergehenden Figuren.

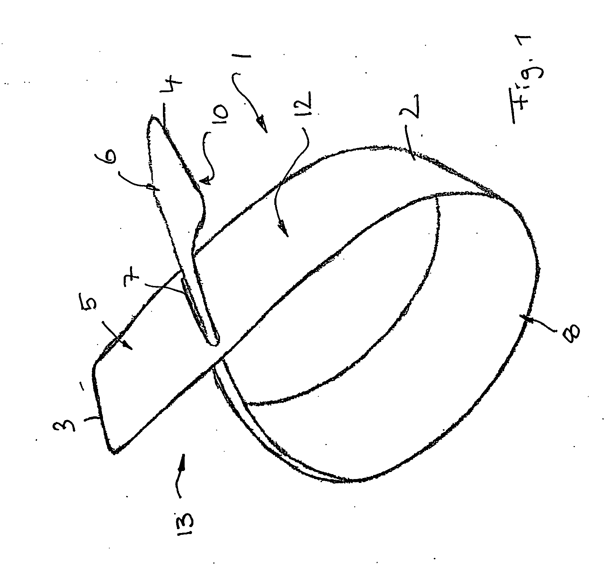

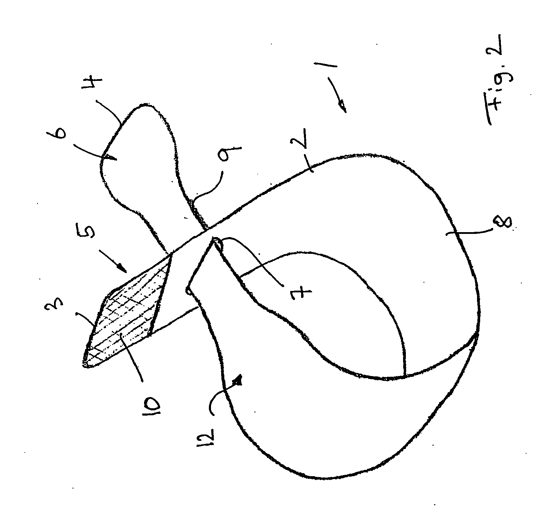

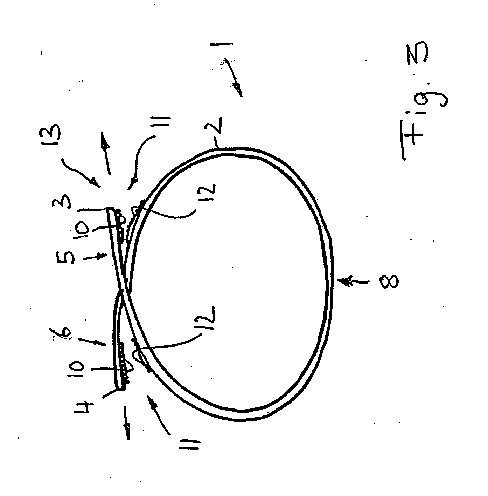

Die in den Figuren gezeichnete Stützbandage 1 umfasst ein zugstabiles, weitgehend dehnungsfreies, jedoch biegsames Gurtband, welches den Bandagegurt 2 bildet. Dieser Bandagegurt 2 umfasst zwei Enden 3 und 4, die sozusagen durcheinander hindurch treten. Wie die Figuren 1 und 2 zeigen, umfasst einer der Endabschnitte 5 einen sich quer zur Gurtlängsrichtung bzw. zur Zugrichtung erstreckenden Durchführschlitz 7, durch den Hindurch der andere Endabschnitt 6 geführt ist, so dass die Enden 3 und 4 des Bandagegurts 2 sozusagen durch sich hindurch treten und beide Enden 3 und 4 auf einer Außenseite der Stützbandage 1 zu liegen kommen, vgl. Fig. 3.

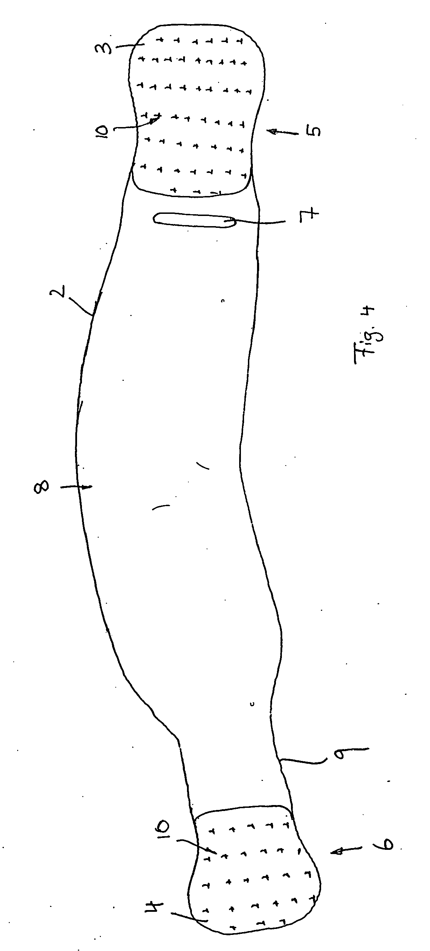

Wie Fig. 4 zeigt, ist ein Mittelabschnitt 8 des Bandagegurts 2 mit einem leicht gekrümmten Verlauf versehen, um die Stützbandagenkontur an die Wölbung eines Mittelfußes anzupassen. Je nach Fußwölbung und/oder zu stützendem Körperglied kann die Wölbung des genannten Mittelabschnitts 8 mehr oder minder stark ausgebildet sein und/oder einen von der in Fig. 3 gezeichneten Ausführung abweichenden Verlauf besitzen. Vorteilhafterweise jedoch kann der Gurtverlauf in der Abwicklung, die Fig. 4 zeigt, hinsichtlich einer Verwölbung im Mittelbereich derart begrenzt sein, dass eine gedachte Verlängerungslinie durch die Enden 3 und 4, die dort mittig angesetzt ist, nicht aus dem Mittelabschnitt des Gurtbandes und der dortigen Kontur hinausläuft, um insgesamt ein straffes Anliegen der Bandage zu ermöglichen.

Wie Fig. 4 zeigt, kann der Bandagegurt 2 in seinem Mittelabschnitt 8 eine im Wesentlichen gleich bleibende Breite besitzen. Zu den Enden 3 und 4 hin kann die Breite des Bandagegurts 2 jedoch abnehmen. Insbesondere kann hierbei vorgesehen sein, dass an dem einen Ende 3, das den genannten Durchführschlitz 7 aufweist, die Breite im Wesentlichen erst hinter dem genannten Durchführschlitz 7 zum Ende 3 hin abnimmt, so dass im Bereich des Durchführschlitzes 7 im Wesentlichen noch die volle Gurtbreite vorhanden ist.

Der gegenüber liegende Endabschnitt 6 hingegen, der durch den Durchführschlitz 7 hindurch geführt ist, kann eine Einschnürung 9 aufweisen bzw. einen Abschnitt verringerter Breite besitzen, in dem die Breite nicht größer als die Länge des Durchführschlitzes 7 ist. Vorteilhafterweise nimmt die Breite des Gurts hinter der Einschnürung 9 zum Ende 4 hin wieder zu, um eine vollflächige Verbindung zu erzielen, die die Zugkräfte auch bei nur kurzer Ausbildung des Endabschnitts 6 sicher überträgt.

In den genannten Endabschnitten 5 und 6 ist der Bandagegurt 2 an seinen Innenseiten, d.h. den Gurtseiten, die zum zu bandagierenden Glied hin gerichtet sind, mit einem Hakenteil 10 einer Haken- und Flauschteilverbindung 11 versehen, wie sie unter dem Handelsnamen Klettverschluss bekannt ist. Wie Fig. 4 zeigt, erstreckt sich der Hakenteil 10 im Wesentlichen über die gesamte Fläche der Endabschnitte 5 und 6.

Dem Flauschteil 12 der Haken- und Flauschteilverbindung 11 bildet die Außenseite des Bandagegurts 2, der im Wesentlichen auf seiner gesamten Außenseite mit Ausnahme der Endabschnitte 5 und 6 als Flauschteil 12 ausgebildet ist.

Hierdurch kann in einfacher Weise die Spannung der Bandage beidseitig, d.h. von beiden Seiten der Schlitzdurchführung her fein justiert werden. Hierzu wird die Stützbandage 1 zunächst um das zu stützende Glied gelegt, so dass die Enden 3 und 4 der Bandage die in den Figuren 1 und 2 gezeigte Stellung einnehmen. Zum Spannen der Stützbandage 1 wird gleichmäßig an beiden Enden 3 und 4 gezogen, so dass diese auseinander gezogen werden, wie dies Fig. 3 zeigt. Liegt die Stützbandage 1 straff an und ist die gewünschte Bandagenspannung erreicht, brauchen lediglich die mit den Händen auseinander gezogenen Enden 3 und 4 auf die als Flauschteil ausgebildete Außenseite der davon überlappten Bandagenabschnitte gedrückt werden, wie dies Fig. 3 verdeutlicht.

## Patentansprüche

1. Stützbandage, vorzugsweise Metatarsalbandage, zur Gewebestützung an einem Körperglied, bestehend aus einem Bandagegurt (2) mit zwei Gurtenden (3, 4) sowie einem Verschluss (13) zum Schließen des Bandagegurts zu einem Ring, **dadurch gekennzeichnet, dass** der Bandagegurt (2) an seinem einen Endabschnitt (5) einen sich quer zur Gurtlängsachse erstreckenden Durchführschlitz (7) aufweist, durch den der andere Endabschnitt (6) des Bandagegurts (2) hindurch geführt ist, und der Verschluss (13) zwei an den Innenseiten der Endabschnitte (5, 6) des Bandagegurts (2) angebrachte Verschlussteile (10) zur Befestigung der Endabschnittinnenseiten auf Außenseiten des Bandagegurts (2) aufweist.

2. Stützbandage nach dem vorhergehenden Anspruch, wobei die beiden Endabschnitte (5, 6) zwei voneinander weg gerichtete Spannabschnitte bilden, die durch Auseinanderziehen voneinander weg die Stützbandage (1) spannen und voneinander weg gerichtet auf der Außenseite von durch die Endabschnitte (5, 6) überlappten Bandagegurtabschnitten befestigbar sind.

3. Stützbandage nach dem vorhergehenden Anspruch, wobei die Verschlussteile (10) derart ausgebildet sind, dass die Endabschnitte (5, 6) durch einfaches Andrücken an die davon überlappten Bandagegurtabschnitte fixierbar sind.

4. Stützbandage nach einem der vorhergehenden Ansprüche, wobei sie frei von Umlenkringen und ähnlichen Umlenkvorrichtungen ausgebildet ist und der Bandagegurt (2) frei von Zugrichtungsumkehrungen ringförmig um das zu stützende jeweilige Körperglied legbar ist, wobei der Bandagegurt (2) an keiner Stelle der Stützbandage mehr als zweilagig übereinander liegt.

5. Stützbandage nach einem der vorhergehenden Ansprüche, wobei die genannten Verschlussteile (10) auf den Endabschnittinnenseiten zusammen mit der Bandagegurtaußenseite einen Haken- und Flauschteilverschluss (11) bilden.

6. Stützbandage nach dem vorhergehenden Anspruch, wobei der Hakenteil (10) der Haken- und Flauschteilverbindung (11) auf der Endabschnittinnenseite und der Flauschteil (12) der Haken- und Flauschteilverbindung (11) auf der Bandagegurtaußenseite vorgesehen ist.

7. Stützbandage nach einem der vorhergehenden Ansprüche, wobei die gesamte Bandagegurtaußenseite, ggf. mit Ausnahme der Endabschnitte (5, 6), als Flauschteil-Verschlussstück (12) ausgebildet ist.

8. Stützbandage nach einem der vorhergehenden Ansprüche, wobei der durch den Durchführschlitz (7) hindurch geführte Endabschnitt (6) einen Abschnitt verjüngter Breite besitzt.

9. Stützbandage nach dem vorhergehenden Anspruch, wobei der Abschnitt verjüngter Breite eine Einschnürung (9) bildet, an die in Gurtlängsrichtung zu beiden Seiten hin Abschnitte größerer Breite anschließen.

10. Stützbandage nach einem der vorhergehenden Ansprüche, wobei die Endabschnitte (5, 6) des Bandagegurts (2) eine kleinere Breite als ein Mittelabschnitt (8) des Bandagegurts (2) besitzen derart, dass die Endabschnitte (5, 6) schmäler als die davon überlappten Bandagegurtabschnitte sind.

11. Stützbandage nach einem der vorhergehenden Ansprüche, wobei der Bandagegurt (2) einen Mittelabschnitt (8) mit einem gekrümmten Verlauf besitzt.
